# FASCICULE DE BREVET EUROPEEN

(11) **EP 0 847 980 B1**
(45) Date de publication et mention de la délivrance du brevet: **06.02.2002**
(21) Numéro de dépôt: 97420227.7
(22) Date de dépôt: 09.12.1997
(51) Int. Cl.: C07C 55/14, C07C 51/42, C07C 51/44, C07C 51/48

(54) **Procédé de traitement de mélanges réactionnels issus de l'oxydation du cyclohexane**
Verfahren zur Aufarbeitung von Reaktionsgemischen die bei der Oxidation von Cyclohexan anfallen
Process for the treatment of reaction mixtures obtained by oxidation of cyclohexane

(30) Priorité: 12.12.1996 FR 9615523
(43) Date de publication de la demande: 17.06.1998
(73) Titulaire: RHODIA POLYAMIDE INTERMEDIATES, 69190 Saint-Fons (FR)
(72) Inventeur: Fache, Eric, 69300 Caluire et Cuire (FR); Leconte, Philippe, 69330 Meyzieu (FR); Marin, Gilbert, 69110 Sainte-foy-les-Lyon (FR)
(74) Mandataire: Esson, Jean-Pierre

(56) Documents cités:
- WO-A-96/03365
- FR-A- 1 266 886
- FR-A- 1 477 314

## Description

La présente invention concerne un procédé d'oxydation du cyclohexane en acide adipique, et plus particulièrement le traitement des mélanges réactionnels issus de cette réaction d'oxydation.

L'oxydation directe du cyclohexane en acide adipique est un procédé qui a été travaillé depuis longtemps, notamment en raison des avantages évidents qu'il y aurait à convertir le cyclohexane en acide adipique, en une seule étape et sans mettre en oeuvre un oxydant tel que l'acide nitrique, ce composé générant des oxydes d'azote qu'il faut ensuite traiter pour éviter toute pollution.

La demande de brevet WO-A-94/07833 décrit l'oxydation d'hydrocarbures cycliques en diacides correspondants, en phase liquide comportant un solvant, à une température d'au moins 60°C, à l'aide d'un gaz contenant de l'oxygène et en présence d'un catalyseur d'oxydation tel qu'un composé du cobalt, en précisant que le solvant représente moins que 1,5 mole par mole d'hydrocarbure cyclique, que ledit solvant comprend un acide organique n'ayant que des atomes d'hydrogène primaires ou secondaires et que la réaction est conduite en présence d'au moins 0,002 mole de catalyseur à base de cobalt pour 1000 g de mélange réactionnel. En fin de réaction, le diacide formé est isolé.

La demande de brevet WO-A-94/07834, déposée le même jour que la précédente, décrit également le même procédé, mais développe les phases de traitement du mélange réactionnel final. Ce traitement consiste à séparer le diacide formé, en refroidissant le mélange pour provoquer la précipitation dudit diacide, à séparer par filtration le diacide de deux phases liquides, une non-polaire qui est recyclée, une polaire qui est également recyclée après une éventuelle hydrolyse et une séparation d'une quantité supplémentaire de diacide.

La demande de brevet WO 96/03365 décrit un procédé équivalent à celui décrit dans les documents cités ci-dessus. Il décrit un procédé de traitement du résidu du milieu réactionnel obtenu après séparation de l'acide adipique produit et des composés volatils. Il concerne un procédé de recyclage du catalyseur.

Ces brevets présentent des solutions permettant d'oxyder en une étape le cyclohexane en acide adipique avec une sélectivité industriellement acceptable, mais ils ne fournissent pas de solution applicable industriellement au traitement du mélange réactionnel issu de l'oxydation, prenant en compte la séparation des différents produits et sous-produits de la réaction, des produits non transformés et du catalyseur.

La présente invention propose un procédé de traitement du mélange réactionnel issu de l'oxydation directe du cyclohexane en acide adipique par l'oxygène, dans un solvant organique et en présence d'un catalyseur, caractérisé en ce que ledit procédé comporte :
- une distillation permettant de séparer d'une part un distillat comprenant au moins une partie des composés les plus volatils tels que le cyclohexane non transformé, le solvant organique, la cyclohexanone, le cyclohexanol, les esters de cyclohexyle, les lactones, l'eau et d'autre part le pied de distillation comprenant les diacides formés, le catalyseur ;
- l'addition d'eau au pied de distillation pour former une solution aqueuse ;
- la cristallisation de l'acide adipique à partir de la solution aqueuse du pied de distillation.

Le solvant organique est plus particulièrement choisi parmi les acides carboxyliques aliphatiques.

Le catalyseur contient de préférence du cobalt, du manganèse ou un mélange de cobalt et de manganèse.

La distillation est conduite de telle façon que la majeure partie et, dans la mesure du possible, la quasi-totalité du cyclohexane non transformé et du solvant, notamment de l'acide carboxylique utilisé de préférence, soit séparée de l'acide adipique.

Le procédé permet ainsi, notamment en raison de la distillation du solvant et plus particulièrement du solvant acide carboxylique, d'opérer la cristallisation de l'acide adipique dans l'eau, solvant qui présente de nombreux avantages par rapport à d'autres solvants de cristallisation tels que l'acide acétique par exemple. En effet, non seulement on évite l'élimination ultérieure souvent difficile de traces de ces solvants, mais également la moindre solubilité dans l'eau à froid de l'acide adipique, permet de limiter, voire, si cela est économiquement acceptable, de supprimer la production d'un deuxième jet d'acide adipique moins pur à partir des solutions provenant de la première cristallisation. En outre dans un procédé industriel, on évite ainsi des risques supplémentaires de corrosion de l'appareillage, apportés par la présence prolongée d'acide carboxylique aliphatique, lorsque celui-ci est utilisé comme solvant.

L'étape de distillation est généralement effectuée à une température de 25°C à 250°C et sous une pression absolue comprise entre 10 Pa et la pression atmosphérique. De préférence la température du mélange pendant la distillation sera maintenue entre 70°C et 150°C.

La distillation peut, si nécessaire, être conduite en plusieurs étapes successives, en particulier dans le mode préféré où l'on souhaite éliminer la plus grosse partie, par exemple plus de 90 % et même plus de 99 % du solvant tel que l'acide carboxylique aliphatique.

De préférence, le mélange réactionnel issu de l'oxydation directe du cyclohexane en acide adipique est soumis à une décantation en deux phases liquides avant l'étape de distillation : une phase supérieure essentiellement cyclohexanique et une phase inférieure comprenant essentiellement le solvant tel qu'un acide carboxylique aliphatique, les diacides formés, le catalyseur et une partie des autres produits de la réaction.

La phase cyclohexanique est le plus souvent réintroduite dans une opération d'oxydation du cyclohexane, soit directement, soit après un éventuel traitement consistant essentiellement à éliminer l'eau qu'elle contient. Ce traitement peut en particulier consister en une distillation azéotropique.

La phase inférieure est soumise à la distillation évoquée précédemment.

Une variante intéressante du procédé de l'invention consiste en l'introduction de vapeur d'eau dans le mélange réactionnel avant ou pendant l'étape de distillation. Cette opération peut permettre de mieux entraîner certains composés présents dans le mélange soumis à la distillation. Elle peut également réaliser une hydrolyse partielle ou complète des esters carboxyliques qui peuvent également se trouver dans le mélange à distiller.

Le distillat obtenu dans l'opération de distillation décrite précédemment comprend les différents composés volatils et de l'eau. Ces composés volatils sont valorisables et sont donc recyclés dans une nouvelle réaction d'oxydation du cyclohexane, après une élimination au moins partielle de l'eau, par tout moyen connu notamment par distillation azéotropique.

L'eau ajoutée au pied de distillation représente de 0,01 à 5 fois le poids du mélange obtenu après ladite distillation. De préférence, la quantité d'eau ajoutée représente de 0,1 à 3 fois ce poids.

Une variante du procédé selon l'invention consiste à effectuer une extraction liquide/liquide sur la solution aqueuse obtenue précédemment, avant de réaliser la cristallisation de l'acide adipique.

Cette extraction est faite à l'aide d'un solvant ou d'un mélange de solvants non miscible à l'eau. A titre d'exemples de tels solvants, on peut citer les hydrocarbures aliphatiques, cycloaliphatiques ou aromatiques comme par exemple l'hexane, le cyclohexane, le benzène, le toluène, les esters comme par exemple l'acétate de butyle, l'acétate de cyclohexyle, les hydrocarbures halogénés comme par exemple le trichlorométhane, les dichlorobenzènes, les éthers comme par exemple le diisopropyléther. Pour son efficacité et pour ne pas compliquer le procédé, on préfère utiliser le cyclohexane. Dans le cadre d'un procédé industriel en marche continue, on pourra utiliser pour cette extraction liquide/liquide tout ou partie de la couche cyclohexanique séparée par décantation du mélange réactionnel, avant l'étape de distillation. Dans le cas préféré de l'utilisation de cyclohexane pour réaliser l'extraction, la solution cyclohexanique obtenue est le plus souvent réintroduite dans une opération d'oxydation du cyclohexane, soit directement, soit de préférence après un traitement consistant essentiellement à éliminer l'eau qu'elle contient. Comme précédemment, ce traitement peut en particulier consister en une distillation azéotropique.

Une autre variante du procédé consiste à chauffer la solution aqueuse obtenue après addition d'eau au pied de distillation, de manière à hydrolyser les esters pouvant être encore présents dans cette solution. Le cyclohexanol formé par cette hydrolyse est séparé par distillation azéotropique. Cette hydrolyse peut être réalisée en présence d'un catalyseur acide fort, soit dissous tel qu'un acide protonique, soit non dissous tel qu'un catalyseur hétérogène acide.

La cristallisation de l'acide adipique à partir de la solution aqueuse est effectuée selon les techniques habituelles de cristallisation. Elle peut être suivie d'une recristallisation de l'acide adipique obtenu si la pureté de ce dernier n'est pas jugée suffisante pour les applications visées.

La solution aqueuse restant après la cristallisation de l'acide adipique contient encore une certaine quantité d'acide adipique dissous que l'on peut récupérer dans un deuxième jet après une concentration de ladite solution aqueuse. Elle contient aussi les autres diacides formés de façon minoritaire lors de l'oxydation du cyclohexane, essentiellement l'acide glutarique et l'acide succinique qui peuvent être séparés par les techniques connues, et enfin le catalyseur. La récupération du catalyseur est effectuée généralement par extraction liquide-liquide ou par électrodialyse membranaire. Le catalyseur ainsi récupéré est recyclé dans une nouvelle réaction d'oxydation du cyclohexane en acide adipique, après ajout d'un complément si nécessaire.

Le mélange réactionnel brut qui est mis en oeuvre dans le procédé de l'invention, provient de l'oxydation connue en soi du cyclohexane, par un gaz contenant de l'oxygène, en milieu liquide comprenant un solvant organique, de préférence un acide carboxylique, et en présence d'un catalyseur, notamment d'un catalyseur contenant du cobalt, du manganèse ou un mélange de cobalt et de manganèse.

Pour la préparation de ce mélange réactionnel brut, on peut se référer aux procédés décrits dans l'art antérieur notamment dans US-A-2 223 493. Ainsi le rapport pondéral initial cyclohexane /acide carboxylique peut se situer par exemple entre 0,1/1 et 10/1 et de préférence entre 0,2/1 et 4/1. Le catalyseur comprend de préférence un composé du cobalt soluble dans le milieu réactionnel, choisi par exemple parmi les carboxylates de cobalt (comme l'acétate de cobalt tétrahydraté), le chlorure de cobalt, le bromure de cobalt, le nitrate de cobalt et/ou un composé du manganèse soluble choisi par exemple parmi les carboxylates de manganèse, (comme l'acétate de manganèse), le chlorure de manganèse, le bromure de manganèse, le nitrate de manganèse.

La quantité de catalyseur, exprimée en pourcentage pondéral de cobalt et/ou de manganèse par rapport au mélange réactionnel, se situe généralement entre 0,01 % et 5 % et de préférence entre 0,05 % et 2 %, sans que ces valeurs soient critiques. Il s'agit cependant d'avoir une activité suffisante tout en n'utilisant pas des quantités trop importantes d'un catalyseur qu'il faut ensuite séparer du mélange réactionnel final et recycler.

Le catalyseur, outre le cobalt et le manganèse, peut également comporter d'autres composés à base de métaux tels que le nickel et/ou le fer et/ou le cuivre et/ou le cérium et/ou le vanadium et/ou l'hafnium et/ou le zirconium.

Il est avantageux de mettre en oeuvre également un composé initiateur de la réaction d'oxydation, tel que par exemple une cétone ou un aldéhyde. La cyclohexanone qui est un intermédiaire réactionnel est tout particulièrement indiquée. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation et lorsque l'on réalise l'oxydation du cyclohexane à une température inférieure à 120°C. Il peut être introduit dès le début de la réaction.

L'acide carboxylique utilisé de préférence comme solvant dans la réaction d'oxydation du cyclohexane est plus particulièrement un acide carboxylique aliphatique saturé ayant de 2 à 9 atomes de carbone et n'ayant que des atomes d'hydrogène primaires ou secondaires.

L'acide acétique est utilisé de préférence comme solvant de la réaction d'oxydation du cyclohexane. Dans la présente description, on se référera parfois par commodité à l'acide acétique comme acide carboxylique utilisé dans les diverses étapes du procédé.

L'oxydation peut également être mise en oeuvre en présence d'eau introduite dès le stade initial du procédé.

La réaction d'oxydation du cyclohexane est généralement effectuée à une température de 60°C à 180°C et de préférence de 70°C à 120°C.

La pression n'est pas un paramètre critique de la réaction et se situe généralement entre 10 kPa (0,1 bar) et 10000 kPa (100 bars).

L'exemple qui suit illustre l'invention.

### EXEMPLE 1

Dans un autoclave de 1,5 litre chemisé en titane et équipé d'une turbine six pales et de diverses ouvertures pour l'introduction des réactifs et des fluides ou pour l'évacuation des produits de la réaction et des fluides, que l'on a préalablement purgé à l'azote, on charge à température ambiante :
- acétate de cobalt tétrahydraté : 4,0 g (16 mmol)
- acide acétique : 359 g (5,98 mol)
- cyclohexane : 289,7 g (3,45 mol)
- cyclohexanone : 3,2 g (32,7 mmol).

Après fermeture de l'autoclave, la pression d'azote est portée à 20 bar, l'agitation est mise en route à 1000 tours/min et la température est amenée à 105°C en 20 min. L'azote est alors remplacé par 20 bar d'air appauvri (5 % d'oxygène). Le débit gazeux en entrée est réglé à 250 litres/heure.

Après une induction de 10 min environ, pendant laquelle il n'y a pas de consommation d'oxygène, la température s'élève de 2 à 3°C et l'oxygène commence à être consommé. Le titre en oxygène de l'air à l'entrée de l'autoclave est progressivement amené à 21 % en fonction de la consommation par l'oxydation.

Le titre en oxygène à la sortie du réacteur reste inférieure à 5 % durant la totalité de l'essai. La température dans l'autoclave oscille entre 104,9 et 105,1°C.

Lorsque 50 litres d'oxygène ont été consommés (taux de transformation du cyclohexane de 20 % environ), on commence l'injection en continu de la phase liquide : injection d'une solution d'acide acétique contenant 1,1 % en poids d'acétate de cobalt tétrahydraté à un débit de 3,7 ml/min et injection de cyclohexane à un débit de 4,1 ml/min. Le produit liquide est stocké en continu dans un décanteur de 7 litres à 70°C.

Après 400 minutes depuis le début de la réaction, l'air est progressivement remplacé par de l'azote, le contenu de l'autoclave est transféré dans le décanteur. Le contenu du décanteur est un mélange biphasique. La phase supérieure, essentiellement cyclohexanique qui contient peu de produits et de cobalt est séparée. La phase inférieure acétique (2164 g) contient l'essentiel des produits de l'oxydation et du cobalt. La phase acétique est soumise à une distillation dans les conditions suivantes :
- pression : 45 kPa, puis 30 kPa
- température : 135°C.

On obtient les résultats rassemblés dans le tableau suivant.

| **Composés** | **Récupéré en tête de distillation** | **Récupéré dans le pied de distillation** |
|---|---|---|
| cyclohexanone | 205 mmol | 0 mmol |
| acétate de cyclohexyle | 22,4 mmol | 0 mmol |
| cyclohexanol | 243,3 mmol | 9 mmol |
| acide glutarique | 0 mmol | 186,4 mmol |
| acide succinique | 0 mmol | 125,3 mmol |
| acide adipique | 0 mmol | 1560 mmol |
| acide hydroxycaproïque | 0 mmol | 94 mmol |
| acide hydroxyadipique | 0 mmol | 76,4 mmol |
| butyrolactone | 80,1 mmol | 24,9 mmol |
| valérolactone | 23,7 mmol | 7,9 mmol |
| acide acétique | 1510 g | < 0,1 mmol |

Le distillat représente 1860 g et le pied de distillation environ 300 g.

On rajoute 1000 g d'eau au pied de distillation. L'ensemble est chauffé à 85°C, puis est progressivement refroidi jusqu'à température ambiante.

Après filtration et lavages à l'eau, on obtient 205 g d'acide adipique brut, ayant une granulométrie moyenne de 300 µm et comportant (en poids par poids) :
- acide succinique : 0,1850 %
- acide glutarique : 0,0020 %
- cobalt : 0,0080 %
- eau : 7 %.

Une recristallisation dans l'eau de cet acide adipique brut conduit à un acide adipique ayant une granulométrie moyenne et comportant (en poids par poids) :
- acide succinique : 0,0002 %
- acide glutarique : < 0,0001 %
- cobalt : < 0,0001 %
- eau : 7 %.

Le catalyseur au cobalt se trouve dans les eaux de cristallisation.

## Revendications

1. Procédé de traitement du mélange réactionnel issu de l'oxydation directe du cyclohexane en acide adipique par l'oxygène, dans un solvant organique et en présence d'un catalyseur, **caractérisé en ce que** ledit procédé comporte :
- une distillation permettant de séparer d'une part un distillat comprenant au moins une partie des composés les plus volatils tels que le cyclohexane non transformé, le solvant organique, la cyclohexanone, le cyclohexanol, les esters de cyclohexyle, les lactones, l'eau et d'autre part le pied de distillation comprenant les diacides formés, le catalyseur ;
- l'addition d'eau au pied de distillation pour former une solution aqueuse ;
- la cristallisation de l'acide adipique à partir de la solution aqueuse du pied de distillation.

2. Procédé selon la revendication 1, **caractérisé en ce que** le mélange réactionnel issu de l'oxydation directe du cyclohexane en acide adipique est soumis à une décantation en deux phases liquides avant l'étape de distillation : une phase supérieure essentiellement cyclohexanique et une phase inférieure comprenant essentiellement le solvant organique, les diacides formés, le catalyseur et une partie des autres produits de la réaction, ladite phase inférieure étant soumise à la distillation.

3. Procédé selon l'une des revendications 1 ou 2, **caractérisé en ce que** l'étape de distillation est effectuée à une température de 25°C à 250°C et sous une pression absolue comprise entre 10 Pa et la pression atmosphérique.

4. Procédé selon l'une des revendications 1 à 3, **caractérisé en ce que** l'on procède à une introduction de vapeur d'eau dans le mélange réactionnel avant ou pendant l'étape de distillation.

5. Procédé selon l'une des revendications 1 à 4, **caractérisé en ce que** l'eau ajoutée après l'étape de distillation représente de 0,01 à 5 fois le poids du mélange obtenu après ladite distillation et de préférence de 0,1 à 3 fois ce poids.

6. Procédé selon l'une des revendications 1 à 5, **caractérisé en ce que** la solution aqueuse obtenue après l'addition d'eau est soumise à une extraction liquide/liquide à l'aide d'un solvant organique non miscible à l'eau.

7. Procédé selon la revendication 6, **caractérisé en ce que** le solvant organique est choisi parmi les hydrocarbures aliphatiques, cycloaliphatiques ou aromatiques, les esters, les hydrocarbures halogénés, les éthers.

8. Procédé selon l'une des revendications 6 ou 7, **caractérisé en ce que** le solvant organique est choisi parmi l'hexane, le cyclohexane, le benzène, le toluène, et est de préférence le cyclohexane.

9. Procédé selon l'une des revendications 1 à 5, **caractérisé en ce que** la solution aqueuse obtenue après l'addition d'eau est soumise à un chauffage, pour hydrolyser les esters encore présents dans cette solution, et le cyclohexanol formé par cette hydrolyse est séparé par distillation azéotropique.

10. Procédé selon la revendication 9, **caractérisé en ce que** l'hydrolyse est réalisée en présence d'un catalyseur acide fort.

11. Procédé selon l'une des revendications 1 à 4, **caractérisé en ce qu'**au moins une partie des composés distillés sont recyclés dans une nouvelle réaction d'oxydation du cyclohexane en acide adipique, après élimination au moins partielle de l'eau qu'ils contiennent.

12. Procédé selon l'une des revendications 1 à 11, **caractérisé en ce que** le solvant organique mis en oeuvre dans l'oxydation du cyclohexane est choisi parmi les acides carboxyliques aliphatiques et est de préférence l'acide acétique.

13. Procédé selon l'une des revendications 1 à 12, **caractérisé en ce que** le catalyseur contient du cobalt, du manganèse ou un mélange de cobalt et de manganèse.

## Claims

1. Method of processing the reaction mixture obtained from the direct oxidation of cyclohexane to adipic acid with oxygen, in an organic solvent and in the presence of a catalyst, **characterized in that** the said method includes:
- a distillation which makes it possible to separate out, on the one hand, a distillate comprising at least some of the most volatile compounds such as the unconverted cyclohexane, the organic solvent, cyclohexanone, cyclohexanol, cyclohexyl esters, lactones and water, and, on the other hand, the distillation residue comprising the diacids formed and the catalyst;
- the addition of water to the distillation residue in order to form an aqueous solution;
- crystallization of the adipic acid from the aqueous solution of the distillation residue.

2. Method according to Claim 1, **characterized in that** the reaction mixture obtained from the direct oxidation of cyclohexane to adipic acid undergoes a separation of the phases by settling into two liquid phases before the distillation step: an upper, essentially cyclohexane phase and a lower phase essentially comprising the organic solvent, the diacids formed, the catalyst and some of the other reaction products, the said lower phase being subjected to distillation.

3. Method according to either of Claims 1 and 2, **characterized in that** the distillation step is carried out at a temperature of from 25°C to 250°C and at an absolute pressure between 10 Pa and atmospheric pressure.

4. Method according to one of Claims 1. to 3, **characterized in that** water vapour is introduced into the reaction mixture before or during the distillation step.

5. Method according to one of Claims 1 to 4, **characterized in that** the water added after the distillation step represents from 0.01 to 5 times the weight of the mixture obtained after the said distillation and preferably from 0.1 to 3 times this weight.

6. Method according to one of Claims 1 to 5, **characterized in that** the aqueous solution obtained after the addition of water is subjected to liquid/liquid extraction using a water-immiscible organic solvent.

7. Method according to Claim 6, **characterized in that** the organic solvent is chosen from aliphatic, cycloaliphatic or aromatic hydrocarbons, esters, halogenated hydrocarbons and ethers.

8. Method according to either of Claims 6 and 7, **characterized in that** the organic solvent is chosen from hexane, cyclohexane, benzene and toluene, and is preferably cyclohexane.

9. Method according to one of Claims 1 to 5, **characterized in that** the aqueous solution obtained after the addition of water is heated, in order to hydrolyse the esters which are still present in this solution, and the cyclohexanol formed by this hydrolysis is separated out by azeotropic distillation.

10. Method according to Claim 9, **characterized in that** the hydrolysis is performed in the presence of a strong acid catalyst.

11. Method according to one of Claims 1 to 4, **characterized in that** at least some of the distilled compounds are recycled in a further oxidation reaction of cyclohexane to adipic acid, after at least partial elimination of the water contained therein.

12. Method according to one of Claims 1 to 11, **characterized in that** the organic solvent used in the cyclohexane oxidation is chosen from aliphatic carboxylic acids and is preferably acetic acid.

13. Method according to one of Claims 1 to 12, **characterized in that** the catalyst contains cobalt, manganese or a mixture of cobalt and manganese.

## Patentansprüche

1. Verfahren zur Behandlung des Reaktionsgemischs, das aus der direkten Oxidation von Cyclohexan zu Adipinsäure mit Sauerstoff stammt, in einem organischen Lösungsmittel und in Gegenwart eines Katalysators, **dadurch gekennzeichnet, dass** besagtes Verfahren umfasst:
- eine Destillation, die es ermöglicht, einerseits ein Destillat abzutrennen, das wenigstens einen Teil der flüchtigsten Verbindungen, wie nicht umgesetztes Cyclohexan, das organische Lösungsmittel, Cyclohexanon, Cyclohexanol, die Cyclohexylester, die Laktone, Wasser, umfasst und andererseits den Destillationsrückstand, der die gebildeten Disäuren und den Katalysator umfasst;
- die Zugabe von Wasser zum Destillationsrückstand, um eine wässrige Lösung zu bilden;
- das Auskristallisieren von Adipinsäure aus der wässrigen Lösung des Destillationsrückstands.

2. Verfahren gemäß Anspruch 1, **dadurch gekennzeichnet, dass** das aus der direkten Oxidation von Cyclohexan zu Adipinsäure hervorgegangene Reaktionsgemisch vor dem Destillationsschritt zu zwei flüssigen Phasen dekantiert wird: eine obere Phase, die im Wesentlichen aus Cyclohexan besteht, und eine untere Phase, die im Wesentlichen das organische Lösungsmittel, die gebildeten Disäuren, den Katalysator und einen Teil der weiteren Reaktionsprodukte umfasst, wobei besagte untere Phase destilliert wird.

3. Verfahren gemäß einem der Ansprüche 1 oder 2, **dadurch gekennzeichnet, dass** der Destillationsschritt bei einer Temperatur von 25 °C bis 250 °C und unter einem Absolutdruck zwischen 10 Pa und Atmosphärendruck ausgeführt wird.

4. Verfahren gemäß einem der Ansprüche 1 bis 3, **dadurch gekennzeichnet, dass** man vor oder während des Destillationsschritts Wasserdampf in das Reaktionsgemisch einführt.

5. Verfahren gemäß einem der Ansprüche 1 bis 4, **dadurch gekennzeichnet, dass** das nach dem Destillationsschritt zugefügte Wasser das 0,01 bis 5fache des Gewichts des nach besagter Destillation erhaltenen Gemischs und vorzugsweise das 0,1 bis 3fache dieses Gewichts darstellt.

6. Verfahren gemäß einem der Ansprüche 1 bis 5, **dadurch gekennzeichnet, dass** die nach der Wasserzugabe erhaltene wässrige Lösung einer Flüssig/Flüssig-Extraktion mit einem nicht wassermischbaren organischen Lösungsmittel unterzogen wird.

7. Verfahren gemäß Anspruch 6, **dadurch gekennzeichnet, dass** das organische Lösungsmittel unter den aliphatischen, cycloaliphatischen oder aromatischen Kohlenwasserstoffen, den Estem, den halogenierten Kohlenwasserstoffen, den Ethern ausgewählt ist.

8. Verfahren gemäß einem der Ansprüche 6 oder 7, **dadurch gekennzeichnet, dass** das organische Lösungsmittel unter Hexan, Cyclohexan, Benzen, Toluen ausgewählt ist und vorzugsweise Cyclohexan ist.

9. Verfahren gemäß einem der Ansprüche 1 bis 5, **dadurch gekennzeichnet, dass** die nach der Wasserzugabe erhaltene wässrige Lösung erhitzt wird, um die in dieser Lösung noch vorhandenen Ester zu hydrolysieren, und das durch diese Hydrolyse gebildete Cyclohexanol durch azeotrope Destillation abgetrennt wird.

10. Verfahren gemäß Anspruch 9, **dadurch gekennzeichnet, dass** die Hydrolyse in Gegenwart eines starken sauren Katalysators durchgeführt wird.

11. Verfahren gemäß einem der Ansprüche 1 bis 4, **dadurch gekennzeichnet, dass** wenigstens ein Teil der destillierten Verbindungen in eine neue Reaktion zur Oxidation von Cyclohexan zu Adipinsäure zurückgeführt wird, nachdem das Wasser, das sie enthalten, wenigstens teilweise entfernt wurde.

12. Verfahren gemäß einem der Ansprüche 1 bis 11, **dadurch gekennzeichnet, dass** das bei der Oxidation des Cyclohexans verwendete organische Lösungsmittel unter den aliphatischen Carbonsäuren ausgewählt ist und vorzugsweise Essigsäure ist.

13. Verfahren gemäß einem der Ansprüche 1 bis 12, **dadurch gekennzeichnet, dass** der Katalysator Kobalt, Mangan oder ein Gemisch aus Kobalt und Mangan enthält.
